## Europäisches Patentamt
## European Patent Office
## Office européen des brevets

(11) Veröffentlichungsnummer: **0 126 379**
**B1**

(12) # EUROPÄISCHE PATENTSCHRIFT

(45) Veröffentlichungstag der Patentschrift:
19.04.89

(51) Int. Cl.⁴: **A 61 K 31/395**, A 61 K 9/18

(21) Anmeldenummer: **84105235.0**

(22) Anmeldetag: **09.05.84**

(54) **Zweiphasenformulierung.**

(30) Priorität: **21.05.83 DE 3318649**

(43) Veröffentlichungstag der Anmeldung:
**28.11.84 Patentblatt 84/48**

(45) Bekanntmachung des Hinweises auf die Patenterteilung:
**19.04.89 Patentblatt 89/16**

(84) Benannte Vertragsstaaten:
**AT BE CH DE FR GB IT LI LU NL SE**

(56) Entgegenhaltungen:
**EP-A-0 047 899
EP-A-0 078 430
DE-A-3 013 839**

**PATENTS ABSTRACTS OF JAPAN, Band 7, Nr. 131
(C-169) 1276 , 8. Juni 1983; & JP - A - 58 46 019
(KANEBO K.K.) 17-03-1983
"Remington's pharmaceutical sciences", 14.
Auflage, 1970, Seite 1706, Mack Publishing Co.,
Easton, Pennsylvania, US.
Drug Development and Industrial Pharmacy, 6(2),
137-160 (1980)**

**Die Akte enthält technische Angaben, die nach
dem Eingang der Anmeldung eingereicht wurden
und die nicht in dieser Patentschrift enthalten sind.**

(73) Patentinhaber: **BAYER AG, Konzernverwaltung RP
Patentabteilung, D-5090 Leverkusen 1 Bayerwerk
(DE)**

(72) Erfinder: **Hegasy, Ahmed, Dr., Tempelhofer
Strasse 57, D-5090 Leverkusen (DE)**
Erfinder: **Rupp, Roland, Dr., Solinger Strasse 18,
D-5653 Leichlingen (DE)**
Erfinder: **Rämsch, Klaus- Dieter, Dr.,
Scheidtstrasse 141, D-5600 Wuppertal 21 (DE)**
Erfinder: **Luchtenberg, Helmut, Dr.,
Windmuehlenstrasse 11a, D-5216 Niederkassel 6
(DE)**

**Beschreibung**

Die Erfindung betrifft Nifedipin enthaltende spezielle feste Arzneizubereitungen, Verfahren zu ihrer Herstellung und ihre Verwendung als Herz/Kreislaufmittel.

Es ist bereits bekannt, daß die Verbindung Nifedipin starke kreislaufbeeinflussende Wirkungen, insbesondere Coronar- und antihypertensive Wirkungen besitzt (vgl. britisches Patent 1 173 862). Aufgrund der schweren Löslichkeit und der hohen Lichtempfindlichkeit von Nifedipin treten bei der galenischen Verarbeitung größere Schwierigkeiten auf, wie aus zahlreichen Publikationen und Patentanmeldungen für spezielle Formulierungen dieses Wirkstoffs ersichtlich wird. Zur Ausnutzung der Coronarwirkung von Nifedipin wurden z. B. Gelatinekapseln hergestellt, die Nifedipin in gelöster Form enthalten und einen schnellen Wirkungseintritt gewährleisten (vgl. US-Patent 3 784 684). Durch diese Kapselformulierung wird der für Coronarerkrankungen notwendige schnelle Wirkungseintritt sichergestellt. Ein Nachteil dieser Zubereitung mit schnellem Wirkungseintritt liegt in der geringen Wirkungsdauer. Bereits 3 Stunden nach der Applikation sinkt die Plasmakonzentration des Nifedipins auf etwa 1/10 der maximalen Anfangskonzentration. Für Langzeitbehandlungen von Coronar- und Hochdruckpatienten ist es daher erforderlich, die Kapsel in kurzen Zeitabständen mehrfach zu applizieren.

Die gleichen Nachteile der kurzen Wirkungsdauer besitzen auch feste Nifedipin-Zubereitungen die z. B. in dem britischen Patent 1 456 618 ("Feste Lösung in Polyethylenglykol") oder in der europäischen Offenlegungsschrift 1 247 beschrieben und beansprucht werden. Auch in der DE-OS-2 822 882 wird versucht die schwere Löslichkeit von Nifedipin durch den Einsatz bestimmter Lösungsvermittler und oberflächenaktiver Substanzen zu kompensieren. Alle diese Nifedipin-Zubereitungsformen besitzen den Nachteil einer geringen Wirkungsdauer. Der Einsatz von zusätzlichen Hilfsstoffen wie Lösungsvermittlern, oberflächenaktiven Substanzen und porösen Trägerstoffen führt häufig zu sehr großen Verabreichungsformen, die vom Patienten nur schwer eingenommen werden können.

Außerdem ist es wünschenswert, die Zahl und die Menge der Hilfs- und Trägerstoffe möglichst niedrig zu halten da bei einem Vergleich zweier Arzneispezialitäten immer das Präparat bevorzugt ist, welches neben dem Wirkstoff möglichst wenig Hilfsstoffe enthält, um unerwünschte biologische Wirkungen weitgehend zu vermeiden.

Es wurde auch versucht, Nifedipin-Zubereitungen mit längerer Wirkungsdauer herzustellen. Diese enthalten Nifedipin in kristalliner Form (vgl. DE-OS-3 039 919). Durch Auswahl einer bestimmten Kristallgröße wird dort versucht, die Freisetzung von Nifedipin in wäßrigen Medien zu verbessern und somit die Absorbierbarkeit und Bioverfügbarkeit bei oraler Applikation zu erhöhen. Die erhaltenen Tabletten zeigen zwar eine deutlich längere Wirkung, die über mehr als 8 Stunden anhält, sie besitzen aber den Nachteil, daß die Wirkung wesentlich später eintritt. Für die akute Behandlung von Coronarerkrankungen ist ein Wirkungseintritt nach wenigen Minuten wünschenswert.

Zur möglichst vollständigen Ausnutzung der vorteilhaften Eigenschaften des Nifedipins ist es ein seit langem bestehendes Bedürfnis, eine einheitliche Zubereitungsform zur Verfügung zu stellen die

a) einen sehr schnellen Wirkungseintritt zeigt, insbesondere zur akuten Behandlung von Coronarerkrankungen;

b) eine lange Wirkungsdauer besitzt, insbesondere zur Behandlung von Hypertonie und zur Dauertherapie;

c) ohne aufwendige galenische Verfahren hergestellt werden kann;

d) als kleine Darreichungsform mit hohem Wirkstoffgehalt vom Patienten sicher und bequem eingenommen werden kann.

Die vorliegende Erfindung betrifft feste zweiphasige Arzneizubereitungen, enthaltend eine Kombination aus einem Nifedipin-Kopräzipitat, in welcher das Nifedipin in gelöster, nicht-kristalliner Form vorliegt und einen kristallinen Nifedipin-Anteil.

Bevorzugt sind feste zweiphasige Arzneizubereitungen wie Tabletten, Dragees, Kapseln oder Sachets, enthaltend eine Kombination aus Nifedipin-Copräzipitat, in welcher 1 Gew.-Teil Nifedipin in 1 bis 10, insbesondere 2 bis 6, Gew.-Teilen Copräzipitatbildner vorliegt, und einen kristallinen Nifedipin-Anteil von 1 bis 5 Gew.-Teilen.

Besonders bevorzugt sind solche feste zweiphasige Arzneizubereitungen in Form von Tabletten oder Dragees.

Als Copräzipitatsbildner sind vorzugsweise geeignet Polyvinylpyrrolidon (PVP), Methylzellulose, Hydroxypropylzellulose und Hydroxypropylmethylzellulose, insbesondere PVP.

Als kristalliner Nifedipinanteil werden vorzugsweise Nifedipinkristalle eingesetzt, die einen durchschnittlichen Partikeldurchmesser von ca. 10 bis 1 µm besitzen, bzw. Nifedipinkristalle, die eine spezifische Oberfläche von 0,5 bis 6 m$^2$/g, insbesondere 1,0 bis 4 m$^2$/g, haben.

Die Herstellung des Nifedipin-Copräzipitats erfolgt durch Auflösung des Nifedipins und des Copräzipitatbildners in einem geeigneten organischen Lösungsmittel. Als Lösungsmittel kommen niedere Chlorkohlenwasserstoffe wie z. B. Methylenchlorid und Chloroform, Aceton und niedere aliphatische Alkohole wie z. B. Ethanol, Isopropanol oder Mischungen davon in Betracht. Nach vollständiger Lösung wird das Lösungsmittel mit Hilfe eines geeigneten Trocknungsverfahrens entfernt (z. B. Vakuumtrocknung, Sprühtrocknung) und das zurückbleibende feste Copräzipitat anschließend zerkleinert.

2

Bei einer Variante dieses Verfahrens kann man auch die Lösung von Nifedipin und Copräzipitatbildner in dem organischen Lösungsmittel direkt mit geeigneten pharmazeutischen Hilfs- und Trägerstoffen weiter granulieren und das organische Lösungsmittel anschließend aus den Granulaten oder Pulvern entfernen, z. B. durch Trocknung.

Als Hilfs- und Trägerstoffe seien beispielhaft genannt: Wasser, pflanzliche Öle (z. B. Erdnuß-/Sesamöl), Alkohole (z. B. Ethylalkohol, Glycerin), Glykole (z. B. Propylenglykol, Polyethylenglykol) , feste Trägerstoffe, wie z B. natürliche Gesteinsmehle (z. B. Kaoline, Tonerden, Talkum, Kreide, Ca-phosphat), synthetische Gesteinsmenle (z. B. hochdisperse Kieselsäure, Silikate), Zucker (z. B. Rohr-, Milch- und Traubenzucker), Emulgiermittel, wie nichtionogene und anionische Emulgatoren (z. B. Polyoxyethylen-Fettsäure-Ester, Polyoxyethylen-Fett-alkohol-Ether, Alkylsulfonate und Arylsulfonate), Dispergiermittel, z. B. Cellulosen oder Cellulosederivate wie Methylcellulose, Natriumcarboxymethylcellulose, Stärke, Lactose, PVP und quervernetztes PVP, und Gleitmittel (z. B. Magnesiumstearat, Talkum, Stearinsäure und Natriumlaurylsulfat).

Die Herstellung des erfindungsgemäß zu verwendenden kristallinen Nifedipins erfolgt durch Mahlen der bei der Synthese erhaltenen Nifedipinkristalle. Diese Mahlung erfolgt vorzugsweise mit Hammermühlen oder Stiftmühlen wobei die gewünschte Partikelgröße erhalten werden kann durch Variation der Drehzahl der Mühle, der zulaufenden Menge des Produktes und/oder der Mahlungsdauer.

Zur Herstellung besonders feiner Nifedipinkristalle (Partikeldurchmesser ca. 1 µm) ist es vorteilhaft Luftstrahlenmühlen einzusetzen.

Die Partikelgröße wird bestimmt über eine Messung der spezifischen Oberfläche nach der Gasadsorptionsmethode (vergl. S. Brunauer: The absorption of gases and vapours, Princeton (1945)).

Bei Kenntnis des Standes der Technik und bei Kenntnis des seit Jahren bestehenden Bedürfnisses, für den schwer zu formulierenden Wirkstoff Nifedipin eine Zubereitungsform zu finden, die sowohl einen schnellen Wirkungseintritt als auch eine lange Wirkungsdauer besitzt, ist es ausgesprochen überraschend, daß durch die erfindungsgemäße Kombination ein sehr einfaches und wirkungsvolles galenisches Prinzip gefunden wurde, welches die Fachwelt in die Lage versetzt, die coronaren und blutdrucksenkenden Wirkungen von Nifedipin optimal auszunutzen. Die nachfolgenden Untersuchungen zeigen, daß die erfindungsgemäße Formulierung in sich gleichzeitig die bekannten positiven Eigenschaften der Nifedipin Gelatinekapsel (schnelles Anfluten des Wirkstoffs) mit der Retardwirkung über mehrere Stunden (langanhaltender Blutplasmaspiegel) vereint.

Fünf gesunden männlichen Probanden wurden jeweils eine Zweiphasentablette gemäß Beispiel 1, enthaltend 10 mg Nifedipin als Copräzipitat und 20 mg kristallines Nifedipin peroral appliziert. Tabelle 1 zeigt die Plasmakonzentrationen (µg/l) von Nifedipin über einen Zeitraum von 8 Stunden.

**Tabelle 1**

Plasmakonzentration (µg/l) nach Applikation der Zweiphasen-Tablette (gemäß Beispiel 1)

| Prob. | 0.00 h | 0.25 h | 0.50 h | 0.75 h | 1.00 h | 1.50 h | 2.00 h | 3.00 h | 4.00 h | 8.00 h |
|---|---|---|---|---|---|---|---|---|---|---|
| 1 | <2 | <2 | 6.43 | 9.07 | 41.49 | 100.35 | 121.35 | 73.79 | 58.98 | 23.81 |
| 2 | <2 | 2.81 | 56.03 | 103.42 | 101.42 | 102.10 | 93.87 | 87.85 | 59.00 | 20.83 |
| 3 | <2 | <2 | 19.14 | 76.74 | 90.96 | 97.76 | 95.36 | 80.12 | 52.68 | 21.83 |
| 4 | <2 | <2 | 16.54 | 51.03 | 115.36 | 133.08 | 126.55 | 92.77 | 80.76 | 33.86 |
| 5 | <2 | 9.32 | 95.06 | 128.20 | 150.22 | 142.00 | 118.46 | 86.52 | 60.78 | 28.33 |
| $\bar{X}$ | <2 | 3.03 | 38.64 | 73.69 | 99.89 | 115.06 | 111.12 | 84.21 | 62.44 | 25.73 |

**Tabelle 2**

Plasmakonzentration (µg/l) nach Applikation einer Tablette mit 20 mg kristalline Nifedipin

| Prob. | 0.00 h | 0.50 h | 1.00 h | 2.00 h | 3.00 h | 4.00 h | 8.00 h | 24.00 h |
|---|---|---|---|---|---|---|---|---|
| 1 | <2 | 10.73 | 16.15 | 16.55 | 28.19 | 29.29 | 11.93 | 7.02 |
| 2 | <2 | 8.11 | 24.75 | 27.34 | 26.84 | 24.04 | 16.62 | 3.20 |
| 3 | <2 | 10.92 | 31.16 | 25.14 | 18.83 | 16.03 | 11.02 | 6.41 |
| 4 | 5.21 | 11.42 | 17.73 | 18.93 | 56.48 | 27.75 | 17.32 | 8.91 |
| 5 | 2.11 | 21.35 | 28.25 | 19.84 | 20.84 | 27.95 | 11.67 | 6.71 |
| $\bar{X}$ | 2.06 | 12.51 | 23.61 | 21.57 | 30.24 | 25.01 | 13.70 | 6.45 |

Dieselbe Gruppe von fünf gesunden Probanden erhielt peroral eine Tablette, welche 20 mg Nifedipin in kristalliner Form enthielt. Die Plasmakonzentrationen zeigen einen verzögerten Wirkungseintritt und insgesamt niedrigere Plasmakonzentrationen (Tabelle 2; s.S. 9).

Die vorstehenden Vergleichsversuche zeigen, daß die erfindungsgemäße kombinierte Zweiphasenformulierung die Vorteile bisher bekannter verschiedener Zubereitungsformen in sich vereinigt ohne deren spezifische Nachteile zu besitzen. Durch diese Formulierung wird der Fachnann in die Lage versetzt, die wertvollen coronar- und blutdrucksenkenden Eigenschaften des Wirkstoffs Nifedipin auszunutzen. Die Zweiphasenformulierung ermöglicht eine einfache, sichere und bequeme Handhabung und stellt eine Bereicherung der Pharmazie dar.

Als Arzneizubereitungen für das kombinierte Zweiphasensystem seien vorzugsweise genannt: Tabletten, Dragees, Granulate, Kapseln, Suppositorien, Sachets und andere feste Arzneizubereitungen. In bestimmten Fällen kann es auch vorteilhaft sein, die erfindungsgemäße Zweiphasenformulierung mit anderen Wirkstoffen zu kombinieren (z. B. mit Betablockern).

Eine vorteilhafte Herstellungsmethode besteht darin, zunächst ein Nifedipin-Copräzipitat in Granulatform herzustellen (Granulat I), in einem zweiten Schritt ein Granulat II, welches kristallines Nifedipin enthält, herzustellen und anschließend die Granulate I und II in dem erfindungsgemäßen Mengenverhältnis zu mischen und in eine geeignete Applikationsform wie z. B. Tabletten oder Dragees zu verpressen oder in Hartgelatinekapseln abzufüllen.

Die folgenden Ausführungsbeispiele erläutern die Erfindung.

## Beispiel 1

### Granulat I:

10 g Nifedipin werden mit 40 g Polyvinylpyrrolidon (PVP) 25 in 60 g Aceton gelöst, mit dieser Lösung wird eine Mischung von 105 g mikrokristalliner Cellulose, 20 g Maisstärke und 10 g quervernetztem PVP granuliert. Die Masse wird im Vakuum getrocknet, anschließend gesiebt und mit 20 g Maisstärke, 14,6 g quervernetztem PVP und 0,4 g Magnesiumstearat gemischt.

### Granulat II:

20 g Nifedipin, 34,8 g mikrokristalliner Cellulose, 12 g Maisstärke und 10 g Milchzucker werden gemischt und mit einem Kleister aus 2 g Maisstärke in Wasser und Zusatz von 1 g Polyoxyethylen Sorbitan Monooleat (Tween 80®) granuliert. Die feuchte Masse wird getrocknet und gesiebt und mit 0,2 g Magnesiumstearat gemischt.

Granulat I und II werden gemischt und eine Menge von 300 mg in Hartgelatinekapseln abgefüllt oder zu Tabletten verpreßt.

## Beispiel 2

### Granulat I:

50 g Nifedipin werden mit 100 g PVP 25 in 350 g Aceton gelöst, mit dieser Lösung wird eine Mischung von 420 g mikrokristalliner Cellulose, 99,5 g Maisstärke und 25 g quervernetztem PVP granuliert. Die Masse wird getrocknet und gesiebt.

### Granulat II:

150 g Nifedipin werden mit 150 g mikrokristalliner Cellulose und 180 g Maisstärke gemischt und mit einem Kleister aus 22,5 g Maisstärke in Wasser granuliert. Die feuchte Masse wird getrocknet und gesiebt.

Granulat I und II werden mit 100 g quervernetztem PVP und 3 g Magnesiumstearat gemischt, und eine Menge von 130 mg in Hartgelatinekapseln abgefüllt oder zu Tabletten verpreßt.

## Beispiel 3

### Granulat I:

100 g Nifedipin werden mit 400 g PVP 25 in 750 g Aceton gelöst, mit dieser Lösung wird eine Mischung von 800 g mikrokristalliner Cellulose, 161 g Maisstärke und 200 g quervernetztem PVP granuliert. Die Masse wird getrocknet und gesiebt.

**Granulat II:**

100 g Nifedipin werden mit 100 g mikrokristalliner Cellulose und 120 g Maisstärke gemischt und mit einem Kleister aus 14 g Maisstärke in Wasser und Zusatz von 1 g Polyoxyethylen Sorbitan Monooleat granuliert. Die feuchte Masse wird getrocknet und gesiebt.

Granulat I und II werden mit 200 g quervernetztem PVP und 4 g Magnesiumstearat gemischt, und eine Menge von 220 mg in Hartgelatinekapseln abgefüllt oder zu Tabletten verpreßt.

**Beispiel 4**

**Granulat I:**

100 g Nifedipin werden mit 1000 g Hydroxypropylmethylcellulose in 800 g eines Lösungsmittelgemisches aus Methylenchlorid und Ethanol gelöst. Das Lösungsmittel wird im Vakuum abgezogen und das zurückbleibende Material zerkleinert.

**Granulat II:**

200 g Nifedipin werden mit 400 g mikrokristalliner Cellulose und 240 g Maisstärke gemischt und mit einem Kleister aus 30 g Maisstärke in Wasser granuliert. Die feuchte Masse wird getrocknet und gesiebt.

Granulat I und II werden mit 126 g quervernetztem PVP und 4 g Magnesiumstearat gemischt und eine Menge von 210 g in Hartgelatinekapseln abgefüllt.

In einer Variante dieses Beispiels werden 210 g dieser Mischung aus den Granulaten I und II zu Tabletten verpreßt.

**Beispiel 5**

**Granulat I:**

100 g Nifedipin werden mit 600 g Hydroxypropylmethylcellulose in 500 g eines Lösungsmittelgemisches aus Methylenchlorid und Ethanol gelöst. Das Lösungsmittel wird im Vakuum abgezogen und das zurückbleibende Material zerkleinert.

**Granulat II:**

300 g Nifedipin werden mit 400 g mikrokristalliner Cellulose, 360 g Maisstärke und 100 g Milchzucker gemischt und mit einem Kleister aus 40 g Maisstärke in Wasser granuliert. Die feuchte Masse wird getrocknet und anschließend gesiebt.

Granulat I und Granulat II werden mit 96 g Natriumcarboxymethylcellulose und 4 g Magnesiumstearat gemischt und eine Menge von 200 g in Hartgelatinekapseln abgefüllt, bzw. zu Tabletten verpreßt.

**Beispiel 6**

**Granulat I:**

50 g Nifedipin werden mit 150 g PVP 25 in 400 g Aceton gelöst, mit dieser Lösung wird eine Mischung von 500 g mikrokristalliner Cellulose, 160 g Maisstärke und 40 g quervernetztem PVP granuliert. Die Masse wird getrocknet und gesiebt.

**Granulat II:**

200 g Nifedipin werden mit 200 g mikrokristalliner Cellulose, 240 g Maisstärke gemischt und mit einem Kleister aus 30 g Maisstärke in Wasser granuliert. Die feuchte Masse wird getrocknet und gesiebt. Granulat I und Granulat II werden mit 126 g quervernetztem PVP und 4 g Magnesiumstearat gemischt und eine Menge von 170 mg in Hartgelatinekapseln abgefüllt, bzw. zu Tabletten verpreßt.

**Beispiel 7**

**Granulat I:**

50 g Nifedipin werden mit 200 g PVP 25 in 400 g Aceton gelöst, mit dieser Lösung wird eine Mischung von 350 g mikrokristalliner Cellulose, 40 g Maisstärke und 25 g quervernetztem PVP granuliert. Die Masse wird getrocknet und gesiebt.

**Granulat II:**

Die Herstellung des Granulats II erfolgt in gleicher Weise wie in Beispiel 2.

Granulat I und II werden mit 129,5 g quervernetztem PVP und 3 g Magnesiumstearat gemischt, und zu Tabletten von jeweils 130 mg Gewicht verpreßt.

**Patentansprüche**

1. Zweiphasige feste Arzneizubereitung enthaltend eine Kombination aus einem Nifedipinkopräzipitat, welches 1 Gewichtsteil Nifedipin in nicht kristalliner gelöster Form und 2 bis 10 Gewichtsteile des Kopräzipitatbildners enthält und einem kristallinen Nifedipinanteil, welcher 1 bis 5 Gewichtsteile Nifedipin enthält.

2. Zweiphasige Arzneizubereitung gemäß Anspruch 1, dadurch gekennzeichnet, daß das Nifedipinkopräzipitat 2 bis 6 Gewichtsteile des Kopräzipitatbildners enthält.

3. Zweiphasige Arzneizubereitung gemäß den Ansprüchen 1 und 2, dadurch gekennzeichnet, daß sie einen Kopräzipitatbildner aus der Gruppe Polyvinylpyrrolidon, Methylcellulose, Hydroxypropylcellulose oder Hydroxypropylmethylcellulose enthält.

4. Zweiphasige Arzneizubereitung gemäß den Ansprüchen 1 bis 3, dadurch gekennzeichnet, daß sie als Koprazipität bildner Polyvinylpyrrolidon enthält.

5. Zweiphasige Arzneizubereitung gemäß den Ansprüchen 1 bis 4, dadurch gekennzeichnet, daß die eingesetzten Nifedipinkristalle eine spezifische Oberfläche von 1,0 bis 4,0 $m^2/g$ haben.

6. Verfahren zur Herstellung von zweiphasigen festen Arzneizubereitungen enthaltend eine Kombination aus einem Nifedipinkopräzipitat, welches 1 Gewichtsteil Nifedipin in nicht kristalliner gelöster Form und 2 bis 10 Gewichtsteile des Kopräzipitatbildners enthält und einem kristallinen Nifedipinanteil, welcher 1 bis 5 Gewichtsteile Nifedipin enthält, dadurch gekennzeichnet, daß man

Nifedipin und den Kopräzipitatbildner in einem organischen Lösungsmittel löst und anschließend das Lösungsmittel entweder sofort oder nach der Granulierung mit geeigneten pharmazeutischen Hilfs- und Trägerstoffen entfernt und dann 1 Gewichtsteil Nifedipin in Form des erhaltenen Nifedipinkopräzipitates mit 1 bis 5 Gewichtsteilen kristallinem Nifedipin mit einer spezifischen Oberfläche von 1,0 - 4,0 $m^2/g$ vermischt und diese Mischung in feste Applikationsformen überführt.

7. Verfahren gemäß Anspruch 6, dadurch gekennzeichnet, daß man als Lösungsmittel für die Herstellung des Nifedipinkopräzipitates niedere Chlorkohlenwasserstoffe mit 1 bis 3 Kohlenstoffatomen, Aceton, niedere aliphatische Alkohole mit 1 bis 4 Kohlenstoffatomen oder Mischungen davon verwendet.

8. Verfahren gemäß den Ansprüchen 6 und 7, dadurch gekennzeichnet, daß man die Lösung des Nifedipins und des Kopräzipitatbildners direkt mit Hilfsstoffen aus der Gruppe kristalline Cellulose, Stärke und quervernetztes PVP granuliert und anschließend das Lösungsmittel entfernt.

**Claims**

1. Two-phase solid medicament formulation containing a combination of a nifedipine coprecipitate, which contains 1 part by weight of nifedipine in a non-crystalline, dissolved form and 2 to 10 parts by weight of the coprecipitate former, and a proportion of crystalline nifedipine, which contains 1 to 5 parts by weight of nifedipine.

2. Two-phase medicament formulation according to Claim 1, characterised in that the nifedipine coprecipitate contains 2 to 6 parts by weight of the coprecipitate former.

3. Two-phase medicament formulation according to Claims 1 and 2, characterised in that it contains a coprecipitate former from the group comprising polyvinylpyrrolidone, methylcellulose, hydroxypropylcellulose or hydroxypropylmethylcellulose

4. Two-phase medicament formulation according to Claims 1 to 3, characterised in that it contains polyvinylpyrrolidone as the coprecipitate former.

5. Two-phase medicament formulation according to Claims 1 to 4, characterised in that the nifedipine crystals employed have a specific surface area of 1.0 to 4.0 m²/g.

6. Process for the preparation of two-phase solid medicament formulation containing a combination of a nifedipine coprecipitate, which contains 1 part by weight of nifedipine in a non-crystalline, dissolved form and 2 to 10 parts by weight of the coprecipitate former, and in proportion of crystalline nifedipine, which contains 1 to 5 parts by weight of nifedipine, characterised in that nifedipine and the coprecipitate former are dissolved in an organic solvent and then the solvent is removed, either immediately or after granulating with suitable pharmaceutical auxiliaries and vehicles, and then 1 part by weight of nifedipine in the form of the resulting nifedipine coprecipitate is mixed with 1 to 5 parts by weight of crystalline nifedipine having a specific surface area of 1.0 - 4.0 m²/g, and this mixture is converted into solid forms for administration.

7. Process according to Claim 6, characterised in that chlorinated lower hydrocarbons having 1 to 3 carbon atoms, acetone, lower aliphatic alcohols having 1 to 4 carbon atoms or mixtures thereof are used as solvent for preparing the nifedipine coprecipitate.

8. Process according to Claims 6 and 7, characterised in that the solution of nifedipine and of the coprecipitate former is immediately granulated with auxiliaries from the group comprising crystalline cellulose, starch and crosslinked PVP, and then the solvent is removed.

**Revendications**

1. Préparation médicamenteuse solide à deux phases comprenant en association un coprécipité de nifédipine, qui contient une partie en poids de nifédipine sous une forme dissoute non cristalline et 2 à 10 parties en poids du coprécipitant, et une fraction cristalline de nifédipine qui contient 1 à 5 parties en poids de nifédipine.

2. Préparation médicamenteuse à deux phases suivant la revendicatioin 1, caractériée en ce que le coprécipité de nifédipine contient 2 à 6 parties en poids du coprécipitant.

3. Préparation médicamenteuse à deux phases suivant les revendications 1 et 2, caractérisée en ce qu'elle contient un coprécipitant du groupe de la popolyvinylpyrrolidone, de la méthylcellulose, de l'hydroxypropylcellulose ou de l'hydroxypropylméthylcellulose.

4. Préparation médicamenteuse à deux phases suivant les revendications 1 à 3, caractérisée en ce qu'elle contient de la polyvinylpyrrolidone code coprécipitant.

5. Préparation médicamenteuse à deux phases suivant les revendications 1 à 4, caractérisée en ce que les cristaux de nifédipine utilisés ont une surface spécifique de 1,0 à 4,0 m²/g.

6. Procédé pour l'obtention de préparations médicamenteuses solides à deux phases comprenant en association un coprécipité de nifédipine, qui contient 1 partie en poids de nifédipine sous une forme dissoute non cristalline et 2 à 10 parties en poids du coprécipitant, et une fraction cristalline de nifédipine qui contient 1 à 5 parties en poids de nifédipine, caractérisé en ce qu'on dissout de la nifédipine et le coprécipitant dans un solvant organique puis on chasse le solvant immédiatement ou après granulation avec des adjuvants et des supports pharmaceutiques appropriés, puis on mélange 1 partie en poids de nifédipine sous la forme du coprécipité de nifédipine obtenu avec 1 à 5 parties en poids de nifédipine cristalline ayant une surface spécifique de 1,0 à 4,0 m²/g et on transforme ce mélange en formes solides d'administration.

7. Procédé suivant la revendication 6, caractérisé en ce qu'on utilise, comme solvants pour l'obtention du coprécipité de nifédipine, des hydrocarbures inférieurs chlorés ayant 1 à 3 atomes de carbone, l'acétone, des alcools aliphatiques inférieurs ayant 1 à 4 atomes de carbone ou des mélanges de ces solvants.

8. Procédé suivant les revendications 6 et 7, caractérise en ce que la solution de nifédipine et de coprécipitant est granulée directement avec des adjuvants du groupe de la cellulose cristalline, de l'amidon et de la PVP à réticulation transversale, puis on chasse le solvant.